# EUROPEAN PATENT SPECIFICATION

(11) **EP 0 996 853 B1**
(45) Date of publication and mention of the grant of the patent: **29.10.2008**
(21) Application number: 97951487.4
(22) Date of filing: 20.11.1997
(51) Int. Cl.: G01F 23/16, G01N 9/26, G01F 23/14, G01F 23/24

(54) **MULTI-SENSOR HYDROSTATIC GAUGE FOR FUEL STORAGE TANKS**
MEHRFACHANORDNUNG ZUR FÜLLSTANDSMESSUNG FÜR KRAFTSTOFF-VORRATSBEHÄLTER
INDICATEUR DE NIVEAU DE CARBURANT HYDROSTATIQUE A CAPTEURS MULTIPLES POUR RESERVOIRS DE CARBURANT

(43) Date of publication of application: 03.05.2000
(73) Proprietor: Innovative Measurement Methods, Inc., Sugar Land, TX 77487 (US)
(72) Inventor: HOBEN, John, Charles, Sugar Land, TX 77479 (US); WESTMORELAND, Allen, Ray, Sugar Land, TX 77479 (US); BUKHMAN, Alexander, 59576 Bat-Yam (IL); BUKHMAN, Israel, 67319 Tel Aviv (IL)
(74) Representative: Cooper, John
(86) International application number: PCT/US1997/021806
(87) International publication number: WO 1999/027344

(56) References cited:
- JP-A- 4 329 315
- US-A- 3 169 396
- US-A- 3 538 746
- US-A- 3 777 177
- US-A- 4 703 664
- US-A- 4 915 507
- US-A- 5 325 716
- US-A- 5 351 725
- PATENT ABSTRACTS OF JAPAN vol. 017, no. 567 (C-1120), 14 October 1993 (1993-10-14) -& JP 05 163651 A (JAPAN VILENE CO LTD), 29 June 1993 (1993-06-29)

## Description

### Field of the Invention

The present invention relates to gauges. More particularly, the present invention relates to gauges for large fuel storage tanks to measure the state properties of the liquids contained therein.

### Background of the Invention

Large storage tanks are used to store hydrocarbon-based fuels for future use. In many cases, tanks are arranged close to one another, forming tank farms. Probes are installed in or onto the tank to monitor both the amount of liquid in the tank and the condition of the liquid itself

Hydrostatic tank gauging ("HTG") systems have been sold commercially since the early 1980's. HTG standards are described in the "Manual of Petroleum Measurement Standards," (American Petroleum Institute, Nov. 1994), specifically, "Chapter 16 - Measurement of Hydrocarbon Fluids by Weight or Mass," incorporated herein by reference for all purposes.

The typical HTG system includes 3 pressure sensors installed along the tank wall. The first sensor is placed near the bottom of the tank. The second sensor is placed about 10 feet above the bottom of the tank. The third sensor is attached to the tank roof.

The first sensor measures hydrostatic pressure of the liquid column and is used for calculating the mass of liquid in the tank. The difference in pressure readings between the first sensor and the second sensor is proportional to the average density of the liquid stored in the tank, thus providing an indication of the level of liquid within the tank. The roof pressure sensor is used for measuring the vapor pressure. The vapor pressure measurement taken by the third sensor is used for compensation of the vapor pressure influence. The typical HTG system also includes a temperature sensor that is installed halfway between the first pressure sensor and the second pressure sensor. The temperature sensor is used to calculate the standard density and standard pressure sensor. The temperature sensor is used to calculate the standard density and standard volume of the liquid, converted back to 60 F (or 15 C).

There are disadvantages with the typical HTG system. These disadvantages include inconvenient and costly installation of the pressure sensors along the tank wall. This installation requires either hot tapping or, in the alternative, taking the tank out of service until the sensors are installed. Further, additional valves must be installed between the tank and the sensor to allow for periodic calibration and maintenance of the pressure sensors.

Another disadvantage of the typical HTG system is that tank bulging affects the measurements. Tank bulging occurs when the walls of the tank deflect under the weight of the liquid contained therein. There is no practical possibility for precise measurement of the distance between the pressure sensors' actual location on the deformed tank wall. Consequently, there is an inherent error in the density calculation, and a corresponding error in the liquid level measurement. This error increases with the age and deterioration of the tank. Furthermore, because the density is measured in the relatively thin layer of liquid, there is more uncertainty in the result, which is calculated as hydrostatic pressure divided by density. Weather factors, such as wind, heating of the sensor by the sun, and temperature differences between environment (sensor body) and liquid (sensor diaphragm), may significantly affect the measured result. Finally, the zero drift of the pressure sensors requires periodic manual calibration and, if not calibrated often, the zero drift is a potential source of error.

Yet another prior art HTG system is sold by Measurement and Control Systems ("MACS"), located at Ackerstein Bldg., 103 Medinat Hayehudim Street, Herzliya Pituach 46766, Israel. The device is called MACStick, and it consists of a probe that is inserted into the middle of the tank The probe itself runs from the bottom through the roof of the tank where the probe is fitted through a collar on the roof to allow for movement of the roof without loss of seal or reference point. The probe consists of a series of pipes that are connected together, end to end, by large flanges. These flanges are welded to the pipes and then are connected to the mating flange of the subsequent pipe, with six or more bolts. The flanges may leak if the bolts connecting the flanges are not torqued evenly or torqued properly. Improper torquing has been a problem in the past and has required additional checking procedures by qualified inspectors. Manufacturing of the flanges also necessitates additional pressure testing, to assure the welding quality critical for a submersible probe construction. Finally, the dozens of nuts and bolts needed to make the necessary connections means that the installation procedure is time consuming and costly.

The MACStick probe also has a special thick flange located between some of the sections. The special thick flange is used as the basis for pressure and temperature sensor installation. The MACStick probe includes a cubical compartment head that is bolted onto the tank roof. The head contains an analog transmitter. The transmitter conveys pressure and temperature signals in 4 to 20 mA current signals. A MACStick analog transmitter can handle two pressure and two temperature sensors. Thus the standard MACStick probe configuration, containing 4 pressure and 4 temperature sensors, must include two analog transmitters in the probe head. In addition, each MACStick probe must have a remote central processing unit ("RCPU") attached to it. The RCPU provides analog to digital conversion of the current transmitters' signals, data processing and calculations.

While useful, the MACStick probe has several disadvantages beyond those mentioned previously. First, most liquid tanks are installed with stilling wells. Stilling wells, typically, are pipes that are fitted within the tank along the centerline. Stilling wells are used for a variety of purposes including the structural integrity of the tank itself. Some stilling wells are perforated to allow liquid from the tank to enter the well. The remaining stilling wells are not perforated. The non-perforated stilling wells are used in jurisdictions having environmental laws that forbid the venting of hydrocarbon vapors into the atmosphere. The MACStick probe, having large flanges positioned at regular intervals, cannot be used in tanks without stilling wells because the seals on the floating roofs cannot operate around the MACStick flanges. However, if the stilling well is not perforated, placing the MACStick probe within the stilling well does no good because the stilling well prevents the liquid from reaching the probe. Consequently, the MACStick probe can only be used on liquid tanks having perforated stilling wells.

In addition to the limited applicability of the MACStick device, there are still other disadvantages. For example, pressure sensors are located within some of the flanges. The pressure sensor location on the flange that has a relatively large diameter means that a rather long channel is needed to provide the surrounding liquid access to the sensor diaphragm. These long channels are easily plugged or clogged by viscous or impure liquids stored in the tanks.

The last significant problem with the MACStick device is its reliance on analog transmitters with limited capacity, particularly when more than four pressure and temperature sensors are required for an application. Finally, MACStick, having analog transmitters, has a much lower tolerance to electromagnetic interference ("EMI") of the analog signal transmission. This design always requires more cables and also requires separate digital processing units for each sensor.

There is, therefore, a need in the art for a probe that is both easy to manufacture, easy to install, and that has the ability to function with floating roofs without violating environmental laws. It is an object of the present invention to provide an easy to make, easy to install, and easy to use probe that will work with any storage tank.

### Summary of the Invention

### Summary of the Invention

This and other objects are achieved by the present invention which is defined in the claims. The probe is composed of sections all having a common outer diameter. The common outside diameter ensures that the present invention can be utilized without stilling wells while allowing standard floating-roof seals that do not violate environmental laws and standards.

In preferred embodiments, the sensor housings and joint sleeves are threadably connected to the various pipe sections. The threadable connections have a double seal using an O-ring and threading (or sealing) paste that ensure a gas-tight seal while being easy to assemble. Pressure and temperature sensors are mounted within the sensor housings. Output leads from the sensors are threaded through the pipe sections and through throughways in the sensor housings and joint sleeves to a circuitry box mounted on the top of the probe rod. The circuitry box contains electronics for processing signals generated by the sensors and for transmitting the results to a remote location.

### Brief Description of the Drawings

A better understanding of the invention will be reached by reading the subsequent detailed description with reference to the drawings wherein:
Fig. 1 shows the overall configuration of the probe of the present invention,
Fig. 2 shows the circuitry elements of the present invention,
Fig. 3 shows a sectional view of a pipe section of the present invention,
Fig. 4 shows a sectional view of the sensor housing of the present invention,
Fig. 5 shows a sectional view of the sensor housing connected to pipe sections of the present invention,
Fig. 6 shows a pressure sensor connected to a sensor housing of the present invention,
Fig. 7 is a sectional view of a joint sleeve of the present invention,

### Detailed Description of the Preferred Embodiments

The present invention is a multi-sensor digital probe having a nearly constant outside diameter that does not interfere with floating roofs of liquid tanks.

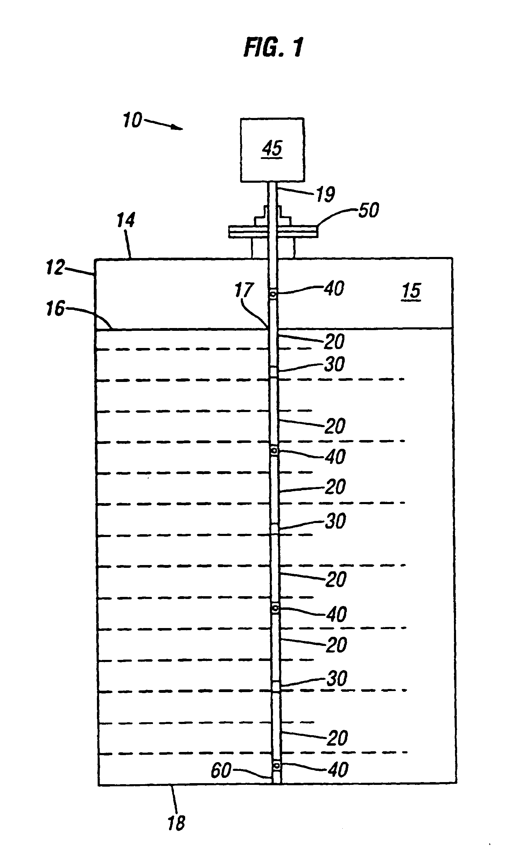

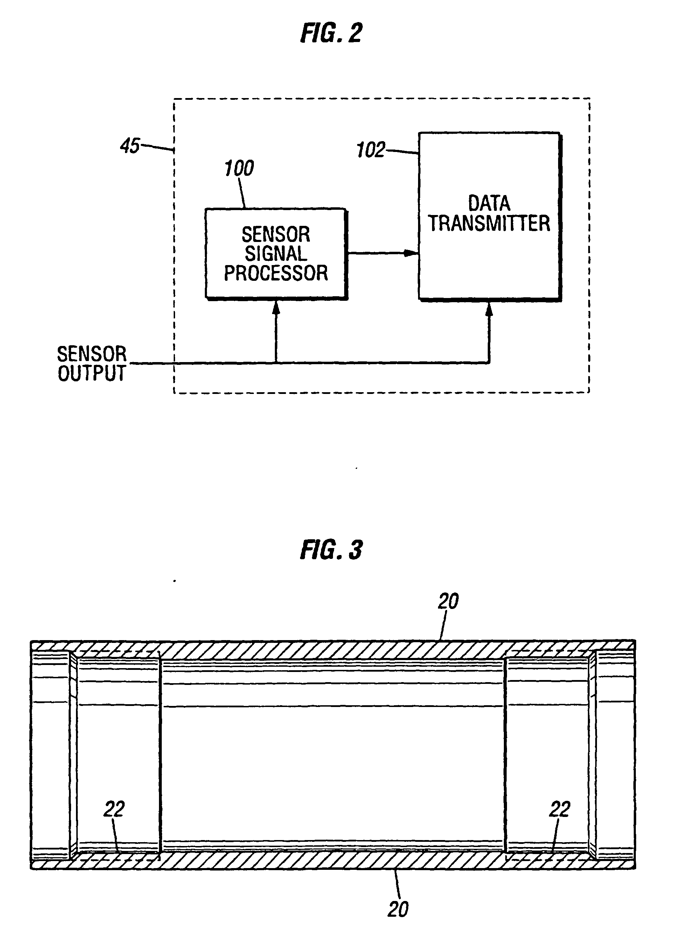

As shown in Fig. 1, the probe 10 has four basic components: pipe sections 20, sensor housings 40, joint sleeves 30, and a circuit box 45. The components are connected in series to form a probe rod 19 with the circuit box 45 at the top of the probe rod 19 as shown in Fig. 1. The pipe sections 20 are standard hollow pipes with female threads 22 at each end as shown in Fig. 3. The threading 22 of the pipe sections is designed to interfit the male threads of the sensor housings 40 and joint sleeves 30.

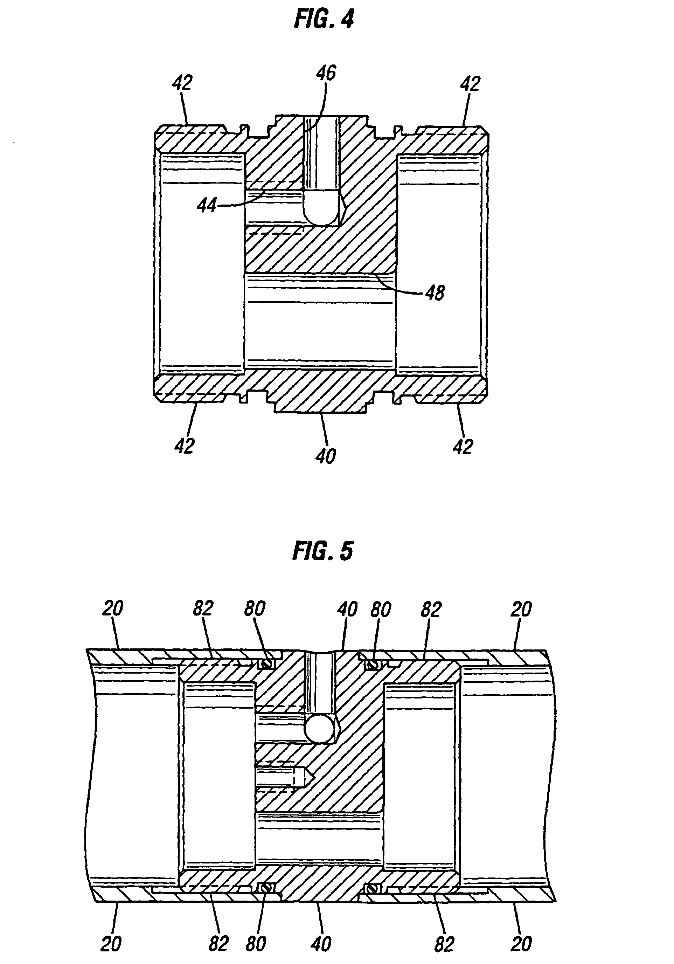

A typical sensor housing is shown in Fig. 4. The sensor housings 40 have male threads 42 at each end so that the sensor housings 40 may be threadably connected to the pipe sections 20. The outer diameter of the sensor housings 40 is the same as the outer diameter of the pipe sections 20. This continuity in outside diameters allows the present invention to be used in liquid tanks 12 having floating roofs 16. With this design, the floating roof can have a constant diameter seal 17 around the probe without having to use stilling wells or to accommodate large flanges as found in the prior art probes. Unlike tanks employing the prior art probes, the liquid tanks utilizing the present invention can use standard floating roof vapor seals that effectively prevent the escape of hydrocarbon gases and allow the liquid tanks to be in compliance with environmental laws.

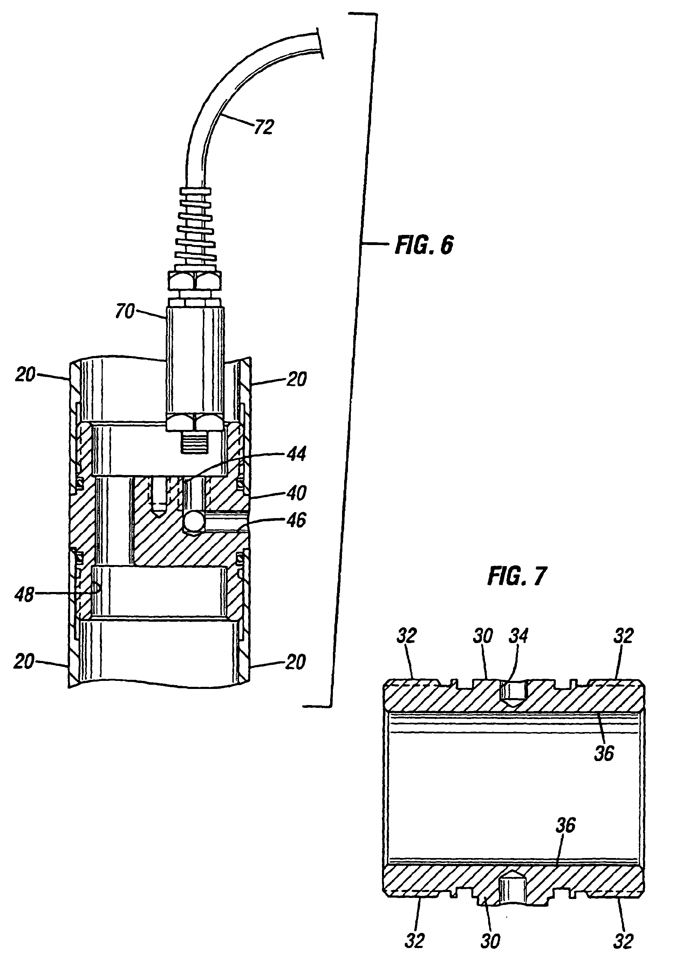

As shown in Fig. 4, the sensor housings 40 have mountings 44 that are designed to hold pressure and/or temperature sensors. A channel 46 is provided in the sensor housing 40 to couple the pressure sensor to the liquid contained in the liquid tank 12. These channels 46 are kept short so as to maximize the free association of surrounding liquid with the pressure sensor and to minimize clogging or liquid dynamic effects that may introduce error into the pressure measurements. Typically, a publicly available pressure sensor (not shown) is used with the present invention. The pressure sensor usually has a diaphragm, and the diaphragm of the pressure sensor is positioned horizontally in the channel 44 in order to provide exact actuation of the diaphragm at that location in the probe rod 19. As shown in Fig. 6, output leads 72 from the temperature and pressure probes 70 in the sensor housing 40, and leads from lower sensor housings, are fitted within a throughway 48 and into the pipe sections 20 leading up to the circuit box 45. No throughway is provided for the bottommost sensor housing 60 that is situated at the floor 18 of the liquid tank 12.

The output of the pressure sensor can be analog or digital. If the pressure sensor, or the temperature sensor output analog signals, then analog-to-digital circuitry 102 should be provided in the circuit box 45 to convert the output signals from the sensors into digital form as shown in Fig. 2. Along with analog-output sensors, the present invention can utilize sensors with digital output. Digital output sensors allow for self-calibration, increased tolerance to EMI, and eliminate the need for analog-to-digital converters. An additional benefit of using sensors having digital output is the ability to measure both gauge pressure and absolute pressure. If absolute pressure readings are needed, at least one sensor must be positioned near the bottom 18 of the liquid tank 12 and one sensor must be placed in the vapor area 15 as shown in Fig. 1. It is a unique feature of the present invention to have pressure and temperature sensors (contained within a sensor housing 40) in the vapor area 15 as shown in Fig. 1.

As with the pressure sensor, a publicly available temperature sensor (not shown) is used. The output of the temperature sensor can be analog or digital. The temperature sensor readings are used for two purposes. First, to measure the temperature of the liquid within the liquid tank. Second, to compensate for the influence of temperature on the pressure sensor measurements.

The pressure and temperature sensors can also be combined into a single unit that can be accommodated within the sensor housing. Combined sensor packages can reduce the number of leads that must be threaded through the probe rod and simplify assembly and maintenance. Furthermore, a combined temperature and pressure sensor package will provide more accurate pressure/temperature characterization results and improve the temperature influence compensation calculations.

Joint sleeves 30 are utilized to join pipe sections 20 where temperature and/or pressure sensors are not needed. As shown in Fig. 7, the joint sleeves 30 have males threads 32 at each end so that the joint sleeves 20 may be threadably connected to the pipe sections 20. The outer diameter of the joint sleeve 20 is the same as the outer diameter of the pipe sections 20. It will be understood that the male-female mating system described herein can be reversed, in that the pipe sections 20 can be fitted with male threads and the sensor housings 40 and joint sleeves 30 fitted with corresponding female threads. As with the sensor housings, the joint sleeves 30 also have throughways 36 to allow sensor output wires to be threaded from the sensors through the probe rod 19 to the circuit box 45. Finally, the joint sleeves 30 can be fitted with side holes 34 that provide a convenient way for work crews to insert and utilize torque-generating tools during assembly and maintenance of the probe rod 19.

The use of threaded connections is an improvement over the prior art that used flanges that were bolted together. As mentioned earlier, the bolts of the prior art devices had to be torqued evenly, otherwise, leaking may occur because part of the sealing O-ring was exposed while another part was over-compressed. In the preferred embodiment of the present invention as shown in Fig. 5, there is a threadable connection between the pipe sections 20 and the sensor housings 40 and joint sleeves 30. Further, the threaded connection of the present invention contains a double seal that is used to seal the connection between the pipe sections 20 and the sensor housings 40 and joint sleeves 30. As shown in Fig. 5, an O-ring 80 is placed within the threaded section to provide a liquid-tight seal. The other part of the double seal consists of thread sealing paste 82 that is applied to the threads when the probe rod 19 is assembled. The threaded connection of the present invention ensures that the O-ring 80 is not subjected to undue stress when the components are assembled. The connection design of the present invention enables easier assembly and a far more reliable gas-tight seal and eliminates many human-errors in the assembly process, a very important consideration for submersible probe design. Unlike the prior art designs, the present invention has only two torque dependent assembly operations instead of the six to twelve torque dependent operations needed to bolt the prior art's flanges together. Furthermore, unlike the prior art assembly methods, the torque dependent assemblies of the present invention do not jeopardize the sealing integrity of the connection. Finally, unlike the prior art designs, there are no welded elements that are a source of leaks and corrosion and that require frequent inspection during construction, assembly, and maintenance.

Yet another benefit of the threaded connection is the ability to use stronger pipes with greater wall thickness than in prior art designs while maintaining the same overall probe diameter. That makes it possible to install the present invention with a wider variety of installation flanges.

One method of assembly of the present invention can be accomplished near the liquid tank itself. The requisite number of the various components, e.g., pipe sections 20, sensor housings 40, sensors, and joint sleeves 30, are provided. To begin, the desired locations of the sensors are identified. The sensor housings 40 are connected to pipe sections 20 to ensure the proper placement of the sensors at the desired locations within the probe rod 19. Pipe sections 20 that are connected by joint sleeves 30 can be used to provide proper spacing between sensor housings 40. Once assembled, the components form a probe rod 19 that is inserted into the liquid tank 12 from a hole 50 in the roof 14 of the liquid tank 12 as shown in Fig. 1. Typically, a bottommost sensor housing 60 is at the tip of the probe rod 19 so that it sits at the bottom 18 of the liquid tank 12. The probe rod 19 rests upon the bottom 18 of the liquid tank 12 and protrudes a short distance from the roof 14 of the liquid tank 12. Optionally, a circuitry box 45 containing circuitry 100 is connected to the probe rod 19. In the preferred embodiment, the circuitry box 45 is threadably connected to the topmost pipe section 20.

An alternate method of assembly of the present invention can be done while situated on the roof of the liquid tank. First pipe sections 20, joint sleeves 30, and sensor housings 40 are provided. Normally, at least one sensor is placed at or near the bottom 18 of the liquid tank 12. To this end, a sensor housing 40 is made the bottommost sensor housing. The wires from the sensors are threaded through a pipe section 30 that is threadably connected to the top side of the bottommost sensor housing 40. The bottommost sensor housing 40 is then inserted into the liquid tank 12 until only a portion of the pipe section 20 protrudes from the roof. At this point, the protruding pipe section is secured and a second sensor housing 40 or joint sleeve 30 is threadably connected to the protruding pipe section 20 to make a topmost sensor housing or joint sleeve. A second pipe section 20 is threadably connected to the last-connected joint sleeve 30 or sensor housing 40 forming a topmost pipe section. The probe is then moved down into the liquid tank 12 until only a portion of the topmost pipe section protrudes from the roof of the tank. This process is repeated until the bottommost sensor housing 40 rests upon the floor 18 of the liquid tank 12 and a portion of the topmost pipe section 20 protrudes from the roof 14 of the liquid tank 12. As with the previous embodiment, a circuitry box 45 is then threadably connected to the topmost pipe section 20.

As shown in Fig. 2, the optional circuitry box 45 contains circuitry 100 for connecting the output leads from the sensors to a remote processor or personal computer (not shown). An alternate embodiment includes circuitry, such as data transmitter 102, for transmitting the output from the sensors to a remote location, by either wires, fiber optics, or wireless methods. While the data transmitter 102 can be analog, the preferred embodiment of the present invention utilizes a digital transmitter. Yet another embodiment includes circuitry, such as sensor signal processor 104, for interpreting the signals, either analog or digital, that are generated by the sensors. The resultant processed data from signal processor 104 can then be transmitted by transmitter 102. As with the data transmitter 102, the preferred embodiment utilizes digital circuitry for the sensor signal processor 104. In the preferred embodiment, both the sensor signal processor 104 and the data transmitter 102 are capable of handling output from multiple sensors, either in sequence or simultaneously.

The foregoing is a description of the arrangement and operation of embodiments of the invention. The scope of the invention is considered to include the described embodiment together with others obvious to those skilled in the art.

## Claims

1. A probe (10) for use in a liquid tank (12) having a floor (18) and walls, capable of continuous measurement of a liquid while immersed in that liquid, wherein said probe (10) comprises:
at least two pipe sections (20), a first section and a second section, each of said pipe sections (20) detachably connectable to the other, wherein each pipe section (20) has an outer diameter which is equal to the outer diameter of the other section, thereby providing a smooth exterior surface when the at least two pipe sections (20) are detachably connected together;
at least one connector (30,40) connecting said pipe sections, said connector constructed and arranged to connect a top end of said first pipe section (20) to a bottom end of said second pipe section (20) to form a probe rod (19) having a common outer diameter, further said connector (30,40) having a channel (44,46) to provide contact with liquid from said liquid tank (12) to one of at least two sensors, a first sensor and a second sensor;
the first sensor, contained in the probe rod (19), capable of continuous temperature measurement, and the second sensor contained in the probe rod (19) capable of continuous pressure measurement through direct contact with the liquid, and wherein said second sensor has a diaphragm which is oriented so that said diaphragm is positioned in use horizontally in said channel (44,46)
means for controlling the first and second sensors to produce output signals; and
means for compiling the output signals and transferring the compiled data to another device.

2. A probe as in claim 1 wherein said at least one connector is a sensor housing (40).

3. A probe as in claim 2, wherein the pressure sensor and the temperature sensor are contained within the sensor housing.

4. A probe as in claim 2 or claim 3, wherein said sensor housing (40) has a throughway (48).

5. A probe as in any preceding claim wherein said at least one further connector is provided in the form of a joint sleeve (30).

6. A probe as in any preceding claim wherein said pipe sections (20) are threaded on said top end and said bottom end.

7. A probe as in any preceding claim, wherein said pressure sensor measures the liquid head above said pressure sensor.

8. A probe as in any preceding claim, wherein said probe (10) further contains circuitry (100), said circuitry (100) capable of calculating compensation factors to compensate for air bubbles locked between the surface of said liquid and said diaphragm of said pressure sensor.

9. A probe as in any preceding claim, wherein said pressure sensor has a digital output.

10. A probe as in any preceding claim, wherein said temperature sensor is capable of measuring the temperature of said liquid in said liquid tank (12).

11. A probe as in claim 10, wherein an output of said temperature sensor is capable of compensating for a temperature influence on a pressure sensor measurement.

12. A probe as in claim 6 wherein said connector (30) has a threaded end (22) capable of interfitting with said threaded ends (22) of said pipe sections (20) to form a threaded connection.

13. A probe as in claim 12, wherein a thread sealing paste is applied to said threaded connection.

14. A probe as in claim 12 or claim 13 wherein an O-ring (80) is positioned within said threaded connection to form a gas tight threaded connection.

15. A probe as in any preceding claim, wherein said probe (10) further has a circuitry box (45).

16. A probe as in claim 15, wherein said circuitry box (45) is positioned at a top of said probe (10).

17. A probe as in claim 16, wherein said circuitry box (45) is threadably connected to one of said pipe sections (20).

18. A probe as in any of claims 15 to 17, wherein said circuitry box (45) contains circuitry (100).

19. A probe as in claim 18, wherein said circuitry (100) is digital.

20. A probe as in claim 18 or claim 19, wherein said circuitry (100) is capable of connecting to another of said probes (10).

21. A probe as in claim 18, wherein said circuitry (100) is capable of interfacing with more than one of said sensors.

22. A probe as in claim 18, wherein said circuitry (100) is capable of interfacing with a processor.

23. A probe as in claim 18, wherein said circuitry (100) is capable of interfacing with a personal computer.

24. A probe as in claim 18, wherein said circuitry (100) contains a sensor signal processor (104).

25. A probe as in any preceding claim, wherein said pressure sensor is self-calibrating.

26. A probe as in any preceding claim, wherein two pressure sensors are provided at spaced locations along the probe to measure absolute pressure.

## Patentansprüche

1. Ein Messfühler (10) zur Verwendung in einem Flüssigkeitsbehälter (12) mit einem Boden (18) und Wänden, der zur kontinuierlichen Messung einer Flüssigkeit imstande ist, während er in dieser Flüssigkeit eingetaucht ist, wobei der Messfühler (10) Folgendes beinhaltet:
mindestens zwei Rohrteilabschnitte (20), einen ersten Teilabschnitt und einen zweiten Teilabschnitt, wobei jeder der Rohrteilabschnitte (20) an dem anderen abnehmbar anschließbar ist, wobei jeder Rohrteilabschnitt (20) einen äußeren Durchmesser aufweist, der gleich dem äußeren Durchmesser des anderen Teilabschnitts ist, wodurch eine glatte Außenoberfläche bereitgestellt wird, wenn die mindestens zwei Rohrteilabschnitte (20) abnehmbar aneinander angeschlossen sind;
mindestens ein Anschlussstück (30, 40), das die Rohrteilabschnitte anschließt, wobei das Anschlussstück konstruiert und angeordnet ist, um ein oberes Ende des ersten Rohrteilabschnitts (20) an einem unteren Ende des zweiten Rohrteilabschnitts (20) anzuschließen, um einen Messfühlerstab (19) mit einem gemeinsamen äußeren Durchmesser zu bilden, wobei das Anschlussstück (30, 40) ferner einen Kanal (44, 46) aufweist, um einem von mindestens zwei Sensoren, einem ersten Sensor und einem zweiten Sensor, Kontakt mit der Flüssigkeit aus dem Flüssigkeitsbehälter (12) bereitzustellen;
den der ersten Sensor, der in dem Messfühlerstab (19) enthalten und zu kontinuierlicher Temperaturmessung imstande ist, und den zweiten Sensor, der in dem Messfühlerstab (19) enthalten und durch direkten Kontakt mit der Flüssigkeit zu kontinuierlicher Druckmessung imstande ist, und wobei der zweite Sensor eine Membran aufweist, die so ausgerichtet ist, dass die Membran bei Gebrauch horizontal in dem Kanal (44, 46) positioniert ist;
Mittel zum Steuern des ersten und zweiten Sensors, um Ausgabesignale zu produzieren; und
Mittel zum Kompilieren des Ausgabesignals und Übertragen der erstellten Daten an ein anderes Gerät.

2. Messfühler gemäß Anspruch 1, wobei das mindestens eine Anschlussstück ein Sensorgehäuse (40) ist.

3. Messfühler gemäß Anspruch 2, wobei der Drucksensor und der Temperatursensor innerhalb des Sensorgehäuses enthalten sind.

4. Messfühler gemäß Anspruch 2 oder Anspruch 3, wobei das Sensorgehäuse (40) einen Durchgang (48) aufweist.

5. Messfühler gemäß einem der vorhergehenden Ansprüche, wobei das mindestens eine weitere Anschlussstück in Form einer Verbindungsmuffe (30) bereitgestellt ist.

6. Messfühler gemäß einem der vorhergehenden Ansprüche, wobei die Rohrteilabschnitte (20) an dem oberen Ende und dem unteren Ende gewindet sind.

7. Messfühler gemäß einem der vorhergehenden Ansprüche, wobei der Drucksensor die Stauhöhe über dem Drucksensor misst.

8. Messfühler gemäß einem der vorhergehenden Ansprüche, wobei der Messfühler (10) ferner eine Schaltung (100) enthält, wobei die Schaltung (100) imstande ist, Kompensationsfaktoren zu berechnen, um Luftblasen zu kompensieren, die zwischen der Oberfläche der Flüssigkeit und der Membran des Drucksensors eingeschlossen sind.

9. Messfühler gemäß einem der vorhergehenden Ansprüche, wobei der Drucksensor eine Digitalausgabe aufweist.

10. Messfühler gemäß einem der vorhergehenden Ansprüche, wobei der Temperatursensor imstande ist, die Temperatur der Flüssigkeit in dem Flüssigkeitsbehälter (12) zu messen.

11. Messfühler gemäß Anspruch 10, wobei ein Ausgang des Temperatursensors imstande ist, einen Temperatureinfluss auf eine Drucksensormessung zu kompensieren.

12. Messfühler gemäß Anspruch 6, wobei das Anschlussstück (30) ein gewindetes Ende (22) aufweist, das imstande ist, zwischen die gewindeten Enden (22) der Rohrteilabschnitte (20) zu passen, um einen Gewindeanschluss zu bilden.

13. Messfühler gemäß Anspruch 12, wobei eine Gewindedichtungspaste auf den Gewindeanschluss aufgetragen wird.

14. Messfühler gemäß Anspruch 12 oder Anspruch 13, wobei ein O-Ring (80) innerhalb des Gewindeanschlusses positioniert wird, um einen gasundurchlässigen Gewindeanschluss zu bilden.

15. Messfühler gemäß einem der vorhergehenden Ansprüche, wobei der Messfühler (10) ferner einen Schaltungskasten (45) aufweist.

16. Messfühler gemäß Anspruch 15, wobei der Schaltungskasten (45) am oberen Ende des Messfühlers (10) positioniert ist.

17. Messfühler gemäß Anspruch 16, wobei der Schaltungskasten (45) an einem der Rohrteilabschnitte (20) gewindet angeschlossen ist.

18. Messfühler gemäß einem der Ansprüche 15 bis 17, wobei der Schaltungskasten (45) eine Schaltung (100) enthält.

19. Messfühler gemäß Anspruch 18, wobei die Schaltung (100) digital ist.

20. Messfühler gemäß Anspruch 18 oder Anspruch 19, wobei die Schaltung (100) imstande ist, an einem anderen der Messfühler (10) angeschlossen zu werden.

21. Messfühler gemäß Anspruch 18, wobei die Schaltung (100) imstande ist, an mehr als einen der Sensoren gekoppelt zu werden.

22. Messfühler gemäß Anspruch 18, wobei die Schaltung (100) imstande ist, an einen Prozessor gekoppelt zu werden.

23. Messfühler gemäß Anspruch 18, wobei die Schaltung (100) imstande ist, an einen PC gekoppelt zu werden.

24. Messfühler gemäß Anspruch 18, wobei die Schaltung (100) einen Sensorsignalprozessor (104) enthält.

25. Messfühler gemäß einem der vorhergehenden Ansprüche, wobei der Drucksensor selbstkalibrierend ist.

26. Messfühler gemäß einem der vorhergehenden Ansprüche, wobei zwei Drucksensoren an beabstandeten Stellen entlang dem Messfühler bereitgestellt sind, um absoluten Druck zu messen.

## Revendications

1. Une sonde (10) destinée à être utilisée dans un réservoir de liquide (12) présentant un plancher (18) et des parois, capable de mesurer en continu un liquide lorsqu'immergée dans ce liquide, ladite sonde (10) comprenant :
au moins deux sections de tuyau (20), une première section et une deuxième section, chacune desdites sections de tuyau (20) étant raccordable l'une à l'autre de façon à ce qu'elles puissent être détachées, chaque section de tuyau (20) ayant un diamètre externe qui est égal au diamètre externe de l'autre section, offrant de ce fait une surface extérieure lisse lorsque ces au moins deux sections de tuyau (20) sont raccordées ensemble de façon à ce qu'elles puissent être détachées ;
au moins un élément de raccord (30, 40) raccordant lesdites sections de tuyau, ledit élément de raccord étant construit et arrangé pour raccorder une extrémité haute de ladite première section de tuyau (20) à une extrémité basse de ladite deuxième section de tuyau (20) afin de former une tige de sonde (19) ayant un diamètre externe commun, ledit élément de raccord (30, 40) présentant de plus un canal (44, 46) pour fournir un contact avec du liquide provenant dudit réservoir de liquide (12) à un capteur parmi au moins deux capteurs, un premier capteur et un deuxième capteur ;
le premier capteur, contenu dans la tige de sonde (19), étant capable de mesurer en continu la température, et le deuxième capteur contenu dans la tige de sonde (19) étant capable de mesurer en continu la pression par contact direct avec le liquide, et ledit deuxième capteur présentant un diaphragme qui est orienté de sorte que ledit diaphragme soit positionné, lors de l'utilisation, de façon horizontale dans ledit canal (44, 46) ;
un moyen destiné à commander les premier et deuxième capteurs afin de produire des signaux de sortie ; et
un moyen destiné à compiler les signaux de sortie et transférer les données compilées dans un autre dispositif.

2. Une sonde telle que dans la revendication 1 dans laquelle ledit au moins un élément de raccord est un logement de capteurs (40).

3. Une sonde telle que dans la revendication 2, dans laquelle le capteur de pression et le capteur de température sont contenus au sein du logement de capteurs.

4. Une sonde telle que dans la revendication 2 ou la revendication 3, dans laquelle ledit logement de capteurs (40) présente un passage traversant (48).

5. Une sonde telle que dans n'importe quelle revendication précédente dans laquelle ledit au moins un élément de raccord supplémentaire est fourni sous la forme d'un manchon de jointure (30).

6. Une sonde telle que dans n'importe quelle revendication précédente dans laquelle lesdites sections de tuyau (20) sont filetées sur ladite extrémité haute et ladite extrémité basse.

7. Une sonde telle que dans n'importe quelle revendication précédente, dans laquelle ledit capteur de pression mesure la charge hydrostatique au-dessus dudit capteur de pression.

8. Une sonde telle que dans n'importe quelle revendication précédente, ladite sonde (10) contenant de plus une circuiterie (100), ladite circuiterie (100) étant capable de calculer des facteurs de compensation pour compenser des bulles d'air bloquées entre la surface dudit liquide et ledit diaphragme dudit capteur de pression.

9. Une sonde telle que dans n'importe quelle revendication précédente, dans laquelle ledit capteur de pression a une sortie numérique.

10. Une sonde telle que dans n'importe quelle revendication précédente, dans laquelle ledit capteur de température est capable de mesurer la température dudit liquide dans ledit réservoir de liquide (12).

11. Une sonde telle que dans la revendication 10, dans laquelle une sortie dudit capteur de température est capable de compenser une influence de la température sur une mesure du capteur de pression.

12. Une sonde telle que dans la revendication 6 dans laquelle ledit élément de raccord (30) présente une extrémité filetée (22) capable de s'imbriquer dans lesdites extrémités filetées (22) desdites sections de tuyau (20) afin former un raccord fileté.

13. Une sonde telle que dans la revendication 12, dans laquelle une pâte d'étanchéité pour raccords filetés est appliquée sur ledit raccord fileté.

14. Une sonde telle que dans la revendication 12 ou la revendication 13 dans laquelle un joint torique (80) est positionné au sein dudit raccord fileté afin de former un raccord fileté étanche aux gaz.

15. Une sonde telle que dans n'importe quelle revendication précédente, ladite sonde (10) présentant de plus un boîtier de circuiterie (45).

16. Une sonde telle que dans la revendication 15, dans laquelle ledit boîtier de circuiterie (45) est positionné au niveau d'un haut de ladite sonde (10).

17. Une sonde telle que dans la revendication 16, dans laquelle ledit boîtier de circuiterie (45) est raccordé par filetage à l'une desdites sections de tuyau (20).

18. Une sonde telle que dans n'importe lesquelles des revendications 15 à 17, dans laquelle ledit boîtier de circuiterie (45) contient de la circuiterie (100).

19. Une sonde telle que dans la revendication 18, dans laquelle ladite circuiterie (100) est numérique.

20. Une sonde telle que dans la revendication 18 ou la revendication 19, dans laquelle ladite circuiterie (100) est capable de se raccorder à une autre desdites sondes (10).

21. Une sonde telle que dans la revendication 18, dans laquelle ladite circuiterie (100) est capable de s'interfacer avec plus d'un desdits capteurs.

22. Une sonde telle que dans la revendication 18, dans laquelle ladite circuiterie (100) est capable de s'interfacer avec un processeur.

23. Une sonde telle que dans la revendication 18, dans laquelle ladite circuiterie (100) est capable de s'interfacer avec un ordinateur personnel.

24. Une sonde telle que dans la revendication 18, dans laquelle ladite circuiterie (100) contient un processeur de signaux de capteurs (104).

25. Une sonde telle que dans n'importe quelle revendication précédente, dans laquelle ledit capteur de pression est à auto-calibrage.

26. Une sonde telle que dans n'importe quelle revendication précédente, dans laquelle deux capteurs de pression sont fournis à des emplacements espacés sur la longueur de la sonde afin de mesurer la pression absolue.
